(19) Europäisches Patentamt
European Patent Office
Office européen des brevets

(11) Publication number: 0 564 006 A2

# (12) EUROPEAN PATENT APPLICATION

(21) Application number: 93108617.7

(51) Int. Cl.[5]: C07D 473/00

(22) Date of filing: 03.05.88

This application was filed on 27 - 05 - 1993 as a divisional application to the application mentioned under INID code 60.

(30) Priority: 04.05.87 YU 786/87
06.11.87 YU 2020/87

(43) Date of publication of application: 06.10.93 Bulletin 93/40

(60) Publication number of the earlier application in accordance with Art.76 EPC: 0 289 992

(84) Designated Contracting States: AT DE FR IT SE

(71) Applicant: KRKA, tovarna zdravil, n.sol.o
Cesta herojev 45
YU-68000 Novo Mesto(YU)
Applicant: KEMIJSKI INSTITUT "BORIS KIDRIC"
Hajdrihova 19
YU-61000 Ljubljana(YU)

(72) Inventor: Kobe, Joze
Pod akacijami 55
YU-61000 Ljubljana(YU)
Inventor: Stimac, Anton
Bratov Ucakar 12
YU-61000 Ljubljana(YU)
Inventor: Zupet, Pavle
Mackuvec 12a
YU-68000 Novo mesto(YU)
Inventor: Gnidovec, Joze
Segova 18
YU-68000 Novo mesto(YU)
Inventor: Plavec, Janez
Lamutova 22
YU-68000 Novo mesto(YU)

(74) Representative: Müller-Boré & Partner
Patentanwälte
Isartorplatz 6
Postfach 26 02 47
D-80059 München (DE)

## (54) Process for preparing purine derivatives and novel purine derivatives.

(57) There are disclosed purine derivatives of the general formula I

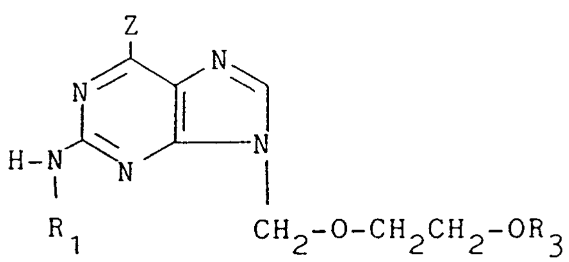

I

wherein
Z = H, $R_1 = CH_3CO$ and $R_3$ = H and further $N^2$-acetyl-9-(2-hydroxyethoxymethyl)guanine also being a compound of the formula I. The preparation of these compounds is also disclosed.

EP 0 564 006 A2

The present invention relates to a process for preparing purine derivatives of the formula I

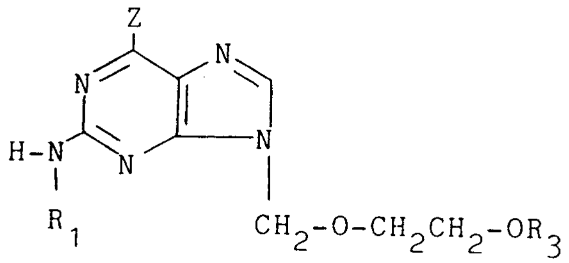

I

wherein

$R_1$ represents hydrogen, $CH_3CO$ or a higher acyl,

$R_3$ represents hydrogen, $CH_3CO$ or a higher acyl, $CF_3CO$, $CCl_3CO$, PhCO or $CH_2$ AR, such as benzyl or substituted benzyl, and benzoisothiazolyl-S,S-dioxide, and

Z represents OH, hydrogen or halo.

Some compounds of formula I are novel compounds and also represent an object of the present invention.

The compounds of the formula I represent - depending upon the different values of the radicals - either intermediates for deoxyacyclovir and acyclovir or deoxyacyclovir (Z = $R_1$ = $R_3$ = H) and acyclovir (Z = OH, $R_1$ = $R_3$ = H) themselves. Acyclovir is the leading medicine for the treatment of herpes infections (Zovirax). Deoxyacyclovir, which is a prodrug of acyclovir, is by means of the enzyme xanthinoxidase quickly converted to acyclovir "in vivo", the "in vivo" activity of the latter being several times greater than the activity of formulated acyclovir (i.e. acyclovir in the form of pharmaceutical formulation).

The synthesis of one of the known compounds of the formula I, i.e. deoxyacyclovir, is disclosed in literature (T.A. Krenitsky et al, Proc. Natl. Acad. Sci., USA (1984) 81, 3209-3213, GB 2 130 204A), preferably via the intermediary compound 2-amino-6-chloro-9-(2-benzoyloxythoxymethyl)purine with dehalogenation and deblocking, or by direct condensation from aminopurine and suitably activated and protected side-chain.

The synthesis of another known compound of formula I , i.e. acyclovir, is disclosed in literature (H.J. Schaeffer et al, (1978) Nature (London) 272, 583-585, US 4,360,522), preferably via guanine.

From the same literature there is also evident the synthesis of the compound I ($R_1$ = H, $R_3$ = $CH_3CO$, Z = OH) from acyclovir. J. Kobe et al (US 4,701,526) also teaches the synthesis of acyclovir from guanine, yet via guanine-glyoxal-adduct to the key intermediate 9-(2-acetoxyethoxymethyl)-glyoxal $N^2$-acetyl-guanine adduct III (R = $R_1$ = $R_3$ = $CH_3CO$).

The process for preparing compounds of the formula I is carried out in such a way that

a) according to the first variant there are prepared the compounds of the formula I, wherein Z represents halo or hydrogen and $R_1$ is hydrogen, from the protected acyclovir of the formula II

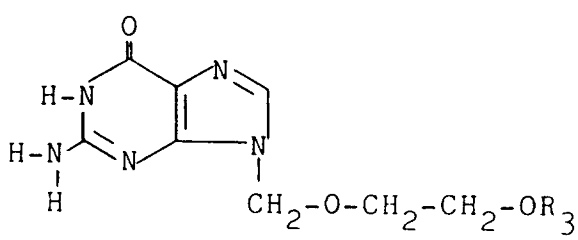

II

wherein $R_3$ has the above meanings except hydrogen, by the reaction thereof with a halogenating agent, such as $POCl_3$, in the presence of tetraethyl ammonium chloride and N,N-dimethylaniline in acetonitrile at reflux, to obtain the compound of the formula I with Z = halo, such as chloro, and $R_3$ having the above meanings except hydrogen, which compound is hydrogenated and, when $R_3$ is different from $CH_2Ar$ or substituted aryl, is deblocked to the compound I with Z = hydrogen and $R_3$ = hydrogen.

The compounds of formula II are prepared according to conventional methods(T.W. Green, Protective Groups in Organic Synthesis), when $R_3$ is different from H. Some of these compounds ($R_3$ is $CF_3CO$,

$CCl_3CO$, benzoisothiazolyl-S,S-dioxide) are novel compounds.

As already mentioned, derivatives of 6-chloropurine (a compound of the formula I, wherein Z = Cl and $R_3$ is different from hydrogen) are by hydrogenation converted to the protected deoxyacyclovir (Z = H, $R_3 \neq$ H, $CH_2Ar$, subst. aryl) and then deblocked ($R_3$ = H) in a weak basic medium, e.g. with ammonia, methylamine (50 %), NaOMe etc., at room temperature. For particular protective groups the deblocking is carried out under conditions disclosed in literature (T.W. Greene, Protective Groups in Organic Synthesis).

When $R_3$ is $CH_2Ar$ or substituted Ar, the hydrogenation alone directly, i.e. without subsequent deblocking, gives unprotected deoxyacyclovir, e.g. catalytic dehydrogenation with Pd/C 5 %.

In the process variant a) according to the invention, the selection of the starting material, i.e. adequately protected acyclovir of the formula II, obtained e.g. for $R_3$ = H according to the process in US 4,701,526, is very convenient. Said protected acyclovir is in a simple way and with good yields converted to known 2-amino-6-chloro-9-(2-benzoyl-oxyethoxymethyl)purine or to novel 2-amino-6-chloro-9-(2-subst. oxyethoxymethyl)purines, which by catalytic hydrogenation may be converted into the prodrug of acyclovir, i.e.deoxyacyclovir.

The process according to the invention may also be carried out in such a way that

b) according to the second variant there are prepared compounds of the formula I with all meanings of radicals as given in the introductory in such a way that a compound of the formula III

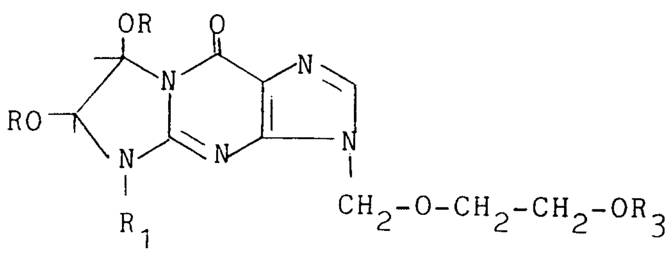

III

wherein R represents $CH_3CO$, isobutyryl or higher acyl and $R_1$ and $R_3$ have the above meanings except hydrogen, is selectively deblocked with weak bases or acids or in the presence of a catalyst to a compound of the formula I, wherein Z is OH, $R_1$ has the meaning at formula I and $R_3$ has the meaning at formula I except hydrogen, which compound is optionally reacted as in the process variant a), i.e. the obtained compound is halogenated to obtain a compound of the formula I, wherein Z is halo, and the latter is, by hydrogenation or photochemical dehalogenation, optionally converted to compound I, wherein Z is hydrogen, or, optionally, a compound of formula I, wherein Z is OH, $R_1$ has the meanings given at formula I and $R_3$ has the meanings given at formula I except hydrogen, benzyl or substituted aryl, is deblocked with a base to a compound of formula I, wherein $R_1$ = $R_3$ = H.

As weak bases or acids diluted mineral and organic acids and bases are suitable.

As catalysts there can be used triethanolamine, methylamine, imidazole, silicagel, molecular sieves, sodium methoxide.

The compound of formula I, wherein Z is OH, $R_1$ is $CH_3CO$ and $R_3$ is H, is a novel compound. Also novel is the compound, wherein Z is H, $R_1$ is $CH_3CO$ and $R_3$ is H. These compounds as well as processes for the preparation thereof are further objects of the invention.

By halogenation of compounds of the formula I, wherein Z is OH, $R_1$ has the meanings at formula I and $R_3$ also has the meanings at formula I except hydrogen, with e.g. $POCl_3$ in the presence or absence of tetraethylammonium chloride and in the presence of N,N-dialkylanilines, preferably diethylaniline, and also of pyridine in acetonitrile at reflux, there is obtained a compound I (Z = Cl), which is by hydrogenation or photochemical dehalogenation converted to protected deoxyacyclovir (Z = H, $R_1$ and $R_3$ have the meanings given at formula I except hydrogen, or one of them should not be hydrogen). Alternatively, compounds of the formula I, wherein Z is OH, $R_1$ has the meanings given at formula I and $R_3$ has the meanings given at formula I except benzyl, substituted aryl or hydrogen, are by deblocking in a basic medium, e.g. with ammonia, methylamine (50 %), NaOMe, NaOH, at room temperature, converted to acyclovir (Z = OH, $R_1$ = $R_3$ = H). In the case of compound I (Z = Cl, $R_1$ = acetyl, $R_3$ = benzyl), for direct synthesis of deoxyacyclovir prodrug or for direct synthesis of deoxyacyclovir there are used hydrogenation technique or other general methods for deblocking under conditions disclosed in literature (T.W. Greene, Protective Groups in Organic Synthesis). Thus the above-mentioned novel compound I with Z = H, $R_1$ = $CH_3CO$, $R_3$ = H, is obtained.

The derivative I ($R_1$ = $R_3$ = $CH_3CO$, Z = OH) can also be selectively converted into compound III ($R_1$ = $CH_3CO$, $R_3$ = H), which is a novel compound as already mentioned above and is also a prodrug. Due to a convenient combination of the meanings of $R_1$ and $R_3$ there exist novel possibilities for further chemical or enzymatic conversions of OH-group as Z in 6-position into other groups, e.g. preferably halo, but also amino, thio, alkylthio, lower alkoxy, azido.

The process variant b) according to the invention makes possible a direct synthesis of the intermediate I, i.e. adequately protected acyclovir, from the compound of formula III disclosed in US 4,701,526, and not from acyclovir (US 4,360,522).

The process variant b) has one step more than a), yet on the other hand, the product represents a pure isomer and according to said variant there are also available two novel compounds as an enrichment of the art.

Some derivatives of formula I are in a simple way and with good yields converted to acyclovir or to already known 2-amino-6-chloro-9-(2-acetoxyethoxymethyl)purine or also to novel 2-amino-6-chloro-9-(2-subst. oxyethoxymethyl)purines, which may subsequently, by means of catalytic hydrogenation or photochemical dehalogenation, be conventrted into a prodrug of acyclovir, i.e. deoxyacyclovir.

The invention is illustrated in detail by the following non-limiting Examples.

Example 1 (variant a)

9-(2-acetoxyethoxymethyl)guanine

9-(2-hydroxyethoxymethyl)guanine (acyclovir) (13.8 g), dry dimethyl formamide (140 ml), acetanhydride (48 ml) and dry pyridine (72 ml) were stirred overnight at room temperature. The suspension was filtered, a white residue was dissolved in hot dimethyl formamide (300 ml), subsequently pyridine (30 ml) and acetanhydride (24 ml) were added and it was stirred for 18 hours at room temprature. White crystals of 9-(2-acetoxyethoxymethyl)guanine were filtered, recrystallized from dimethyl formamide, washed with ethyl acetate and dried in vacuo at 110°C.

Yield:10.04 g (61 %), m.p. 234-245°C.

Example 2 (variant a)

9-(2-acetoxyethoxymethyl)guanine

9-(2-hydroxyethoxymethyl)-$N^2$-acetylguanine (150 mg, 0.5 mmole) was heated under stirring at reflux for 7 hours in methanol (5 ml) with silicagel (0.5 g; Kieselgel 60 $HF_{254}$, Merck), previously dried at 110°C for 1 hour. The boiling suspension was filtered through Celite and washed with hot methanol (25 ml). After evaporation crude 9-(2-acetoxyethoxymethyl)guanine (75 mg; 57 %) was obtained, m.p. 241-242°C.

Example 3 (variant b)

9-(2-acetoxyethoxymethyl)guanine

To a solution of 9-(2-acetoxyethoxymethyl)-diacetoxy-glyoxal-$N^2$-acetylguanine-adduct of the formula III (R = $R_1$ = $R_3$ = $CH_3CO$; 0.5 g, 1.1 mmole) with the chemical name 5-acetyl-6,7-diacetoxy-3-(2-acetoxyethoxymethyl)-5 6,7,9-tetrahydro-3H-imidazo/1,2-a/purine-9-one in hot methanol (20 ml) there was added triethanolamine (1.64 g, 11 mmole) and the reaction mixture was heated at reflux for 5 hours. After cooling to room temperature 9-(2-acetoxyethoxymethyl)guanine (75 g) of the formula I ($R_1$ = H, $R_3$ = $CH_3CO$, Z = OH) was obtained. The mother liquor was evaporated and water (5 ml) was added to the residue. There was obtained the compound of the formula I (100 mg; $R_1$ = H, $R_3$ = $CH_3CO$, Z = OH). The total yield was 175 mg (59 %), m.p. 240-242°C. The product was identical with the compound disclosed in U.S. patent 4,360,522.

Example 4 (variant b)

$N^2$-acetyl-9-(2-acetoxyethoxymethyl)guanine

To a solution of 9-(2-acetoxyethoxymethyl)-diacetoxyglyoxal--$N^2$-acetylguanine-adduct of the formula III (R = $R_1$ = $R_3$ = $CH_3CO$; 45 mg, 0.1 mmole) in hot methanol (2 ml) 2 drops (~50 mg, 0.3 mmole) of

triethanolamine were added and the mixture was heated for 70 minutes at reflux. Water (1 ml) was added to the evaporated mixture. A compound of m.p. 203-205°C10 mg; 33 %) was obtained, which was identified as $N^2$-acetyl-9-(2-acetoxyethoxymethyl)guanine of the formula I ($R_1$ = $R_3$ = $CH_3CO$, Z = OH).
$^1$H NMR - DMSO-$d_6$, δ-TMS
1.95 (s, 3, OCO Me)
2.17 (s, 3, NNCO Me)
3.71 (m, 2, $OCH_2\overline{CH_2}$)
4.06 (m, 2, $O\overline{CH_2}CH_2$)
5.46 (s, 2, $NCH_2O$)
8.10 (s, 1, $H_8$)
11.7 (broad. s, NH)
12.0 (broad. s, NH)
The aqueous liquor was evaporated and the residue (yellow oil) was re-dissolved in hot methanol. After cooling there was obtained 9-(2-acetoxyethoxymethyl)guanine (10 mg; 38 %) of the formula I ($R_1$ = H, $R_3$ = $CH_3CO$, Z = OH).

Example 5 (variant b)

9-(2-acetoxyethoxymethyl)guanine

a) $N^2$-acetyl-9-(2-acetoxyethoxymethyl)guanine (15.5 g, 0.05 mole) and triethanolamine (7.5 g,0.05 mole) were heated at reflux for 2.5 hours under stirring in methanol (750 ml). The reaction mixture was left to cool to room temperature, the obtained crystals were filtered and washed with methanol (cca 100 ml) to a neutral pH. After air-drying there was obtained 9-(2-acetoxyethoxymethyl)guanine (12.23 g; 92 %), m.p. 242-244°C (H.J. Schaeffer, US 4,287,188).
$^1$H NMR - DMSO-$d_6$, δ-TMS
1.95 (s, 3, $COCH_3$)
3.66 (m, 2, $OCH_2CH_2OAc$)
4.05 (m, 2, $O\overline{CH_2}CH_2OAc$)
5.34 (s, 2, $NCH_2O$)
6.49 (broad s, 2, $NH_2$)
7.79 (s, 1, $H_8$)
10.65 (broad s, 1, NH)

| Elemental analysis: | | | |
|---|---|---|---|
| Calc.: | C 44.94 | H 4.90 | N 26.20 |
| Found: | C 45.00 | H 4.90 | N 26.39 |

Example 6 (variant b)

9-(2-acetoxyethoxymethyl)-2-amino-9H-purine

a) The crude residue (1.08 g) from the reaction of the example 10 hereinafter containing 9-(2-acetoxyethoxymethyl)-2-amino-6-chloro-9H-purine (0.5 g) was dissolved in absolute ethanol (100 ml), triethylamine (1 ml) and 10 % Pd on charcoal (0.5 g) were added and hydrogenation was carried out for 15 hours in a Parr hydrogenator at an initial pressure of 350000 Pa (50 psi) and at room temperature. The mixture was filtered and the ethanolic solution was evaporated to a solid residue, which was extracted with boiling benzene (3 x 50 ml). The combined extracts were evaporated to an oily residue, from which the product was isolated by chromatography on silicagel (80 g; 70 to 230 mesh). First the impurities were washed with etyhl acetate (cca 2 l) and then 9-(2-acetoxyethoxymethyl)-2-amino-9H-purine (0.30 g, 68 %) was washed with acetone (cca 1 l).
b) 9-(2-acetoxyethoxymethyl)-2-amino-6-chloro-9H-purine (71 mg, 0.25 mmole) was dissolved in dry tetrahydrofuran (18 ml) and triethylamine (2 ml). The solution was flushed with nitrogen for 10 minutes and then photolyzed in a photo-chemical reactor Rayonet (254 nm). The volatile components were evaporated, the residue was dissolved in chloroform (5 ml) and washed with water (3 x 40 ml). The organic layer was dried with anhydrous sodium sulfate and the solvent was evaporated. There was

obtained crude 9-(2-acetoxyethoxymethyl)-2-amino-9H-purine (34mg), identical to the product obtained by method a).

Example 7 (variant b)

2-amino-9-(2-hydroxyethoxymethyl)-9H-purine (DEOXYACYCLOVIR)

9-(2-acetoxyethoxymethyl)-2-amino-9H-purine (0.3 g) was stirred for 1 hour with a 40 % aqueous methylamine solution (2 ml) and ethanol (5 ml). The volatile components were evaporated to a solid residue and crystallized from ethanol. There was obtained pure 2-amino-9-(2-hydroxyethoxymethyl)-9H-purine (102 mg, 41 %), m.p. 189-192°C.

$^1$H NMR - DMSO-$d_6$, $\delta$-DSS
3.50 (s, 4, $CH_2CH_2$)
4.66 (s, 1, OH)
5.45 (s, 2, $NCH_2O$)
6.53 (s, 2, $NH_2$)
8.13 (s, 1, $H_8$)
8.56 (s, 1, $H_6$)

Example 8

A. A mixture of 9-(2-acetoxyethoxymethyl)-$N^2$-acetyl guanine (618 mg, 2 mmole), silicagel powder (2.50 g; Kieselgel 60 $HF_{254}$, Merck) and methanol (30 ml) was vigorously stirred at reflux for 8 hours. The solvent was evaporated and the resulting solid was boiled for 1 minute with dimethyl formamide (30 ml). The suspension was filtered through Celite, the layer of silica was extracted with boiling dimethyl formamide (10 ml), filtered through Celite and washed with hot solvent (5 ml). Combined filtrates were evaporated to yield 9-(2-acetoxyethoxymethyl)guanine (515mg, 96 %), m.p. 242-244°C, which is identical with the product obtained in method A. The crude product contained max. 10 % of acyclovir according to HPLC analysis.

B. A mixture of 9-(2-acetoxyethoxymethyl)-$N^2$-acetyl guanine (618 mg, 2 mmole), molecular sieve 0.3 nm powder (618 mg, Molekularsieb 3 Å Pulver, Merck) and methanol (30 ml) was vigirously stirred at reflux for 2 hours. The solvent was evaporated, the resulting solid was heated in dimethyl formamide (30 ml) to the boiling point, quickly filtered through Celite and washed with hot dimethyl formamide (2 X 5 ml). The filtrate was evaporated to yield 9-(2-acetoxyethoxymethyl)guanine (534 mg, 100 %), m.p. 238-242°C, which is identical with the product obtained in method A. The crude product contained about 15 % of acyclovir according to HPLC analysis.

C. A mixture of 9-(2-acetoxyethoxymethyl)-$N^2$-acetyl guanine (1.55 g, 5 mmole), imidazole (0.34 g), methanol (30 ml) and water (10 ml) was stirred at reflux for 16 hours. The obtained crystalline product was filtered while hot, successively washed with methanol, water and methanol, and dried to give 9-(2-acetoxyethoxymethyl)guanine (0.65 g, 48 %), m.p. 242-243°C, which is identical with the product obtained in method A. The crude product contained max. 6 % of acyclovir according to HPLC analysis.

Example 9

$N^2$-acetyl-9-(2-hydroxyethoxymethyl)guanine

To a freshly prepared solution of sodium (0.92 g) in dry methanol (50 ml) there was added 9-(2-acetoxyethoxymethyl)-$N^2$-acetyl guanine (6.18 g, 20 mmole). The solution was stirred at 20°C for 16 minutes and then the reaction was quenched by the addition of a 5 % HCl solution (30 ml). Upon cooling, a white crystalline mass separated and it was allowed to stand overnight, filtered and successively washed with water, ethanol and ether. Upon drying there was obtained $N^2$-acetyl-9-(2-hydroxyethoxymethyl)guanine (4.19 g, 78 %), which contained less than 4 % of acyclovir according to HPLC analysis. This sample had a m.p. 214-215°C (unchanged after crystallization from water).

$^1$H NMR - DMSO-$d_6$ $\delta$-TMS:
2.18 (s, 3, $COCH_3$)
3.49 (m, 4, $CH_2\overline{C}H_2$)
4.66 (br, 1, OH)
5.48 (s, 2, $OCH_2$Base)

8.13 (s, 1, $H_8$)
11.65, 11.95 (two broad s, NH's)
ms (FAB) m/z: 268 ($MH^+$), ms (EI) m/z: 267.097 (M + ($C_{10}H_{13}N_5O_4$) = 267.09675)

| Elemental analysis: | | | |
|---|---|---|---|
| Calc.:<br>Found: | C 44.94<br>C 45.32 | H 4.90<br>H 5.05 | N 26.20<br>N 25.72 |

Example 10

9-(2-acetoxyethoxymethyl)-2-amino-6-chloro-9H-purine

A solution of 9-(2-acetoxyethoxymethyl)guanine (4.01 g, 15 mmole), tetraethylammonium chloride (4.98 g, 30 mmole; dried in vacuo at 85°C overnight over $P_2O_5$), N,N-dimethylaniline (1.92 ml, 15 mmole; dried over and distilled from $CaH_2$) and freshly distilled phosphorus oxychloride (8.4 ml, 90 mmole) in acetonitrile (30 ml) was heated with stirring at reflux for 15 minutes. The volatile materials were evaporated in vacuo, the resulting oil was dissolved in chloroform (90 ml) and vigorously stirred with broken ice for 15 minutes. The layers were separated and the aqueous pahse was extracted with chloroform (6 x 50 ml). The combined organic phase was washed with cold water (6 x 50 ml) and 5 % $NaHCO_3/H_2O$ (50 ml), dried over $Na_2SO_4$ overnight and filtered. The evaporation gave a crude product (2.06 g), which was applied to a silicagel column and eluted first with chloroform (1 l) to remove N,N-dimethylaniline and then with ethyl acetate. The fractions containing the required product free of by-products (TLC in EtOAc: $R_f$ = 0.35) were combined and evaporated to give 9-(2-acetoxyethoxymethyl)-2-amino-6-chloro-9H-purine (0.88g, 21 %) with m.p. 104-105°C after recrystallization first from ethyl acetate and then from 2-propanol.
$^1H$ NMR ($CDCl_3$) δ-TMS:
2.04 (s, 3, $COCH_3$)
3.76 ($A_2B_2$m, 2, $\overline{O}CH_2CH_2OAc$)
4.21 ($A_2B_2$m, 2, $O\overline{C}H_2CH_2OAc$)
5.53 (s, 2, $OCH_2$Base)
5.63 (br, 2, $NH_2$)
7.93 (s, 1, $H_8$)
ms m/z: 285 ($M^+$)

Example 11

9-(2-acetoxyethoxymethyl)-2-acetamido-6-chloro-9H-purine

A solution of 9-(2-acetoxyethoxymethyl)-$N^2$-acetylguanine (3.09 g, 10 mmole), N,N-diethylaniline (1.59 ml, 10 mmole) and freshly distilled phosphorus oxychloride (9.6 ml) in acetonitrile (40 ml) was heated with stirring at reflux for 30 minutes. The volatile materials were evaporated in vacuo, ice (50 g) was added to the residue and it was extracted with chloroform (10 x 30 ml). The combined organic phase was washed with cold water (3 x 75ml and 5 % $NaHCO_3/H_2O$ (100 ml), dried over $Na_2SO_4$ for 1 hour and filtered. The evaporation gave an orange viscous substance (1.31 g), which was applied to a silicagel column and the column was eluted with acetone (300 ml). A crude product (0.67 g) was obtained, which was repurified on a second silicagel column and eluted with 1:1 benzene:ethyl acetate to yield 9-(2-acetoxyethoxymethyl)-2-acetamido-6-chloro-9H-purine (0.47 g, 14 %) with m.p. 150-150.5°C after recrystallization from ethyl acetate.
$^1H$ NMR - DMSO-$d_6$, δ-TMS:
1.96 (s, 3, $OCOCH_3$)
2.26 (s, 3, $NHCO\overline{C}H_3$)
3.83 ($A_2B_2$m, 2, $O\overline{C}H_2CH_2OAc$)
4.13 ($A_2B_2$m, 2, $O\overline{C}H_2CH_2OAc$)
5.68 (s, 2, $OCH_2$Base)
8.69 (s, 1, $H_8$)
10.75 (br, 1, NH)

Example 12

9-(2-acetoxyethoxymethyl)-2-amino-9H-purine

To a solution of 9-(2-acetoxyethoxymethyl)-2-amino-6-chloro-9H-purine(7.15 g, 25 mmole) in acetone (100 ml) there were succesively added triethylamine (35 ml, 250 mmole), formic acid (5 ml, 125 mmole) and 10 % palladium on charcoal (1 g). The mixture was heated with vigorous stirring at reflux for 1 hour, filtered while hot and the catalyst was washed with boiling acetone. The filtrate was evaporated to dryness, the residue was dissolved in water (100-150 ml) and the solution was continuously extracted with dichloromethane overnight. The organic layer was dried over $Na_2SO_4$ for cca 1 hour and evaporated to dryness to give a crude product (6.25 g). It was recrystallized from 100 % ethanol to give 9-(2-acetoxyethoxymethyl)-2-amino-9H-purine (5.51 g, 88 %) with m.p. 133-133.5°C.

$^1$H NMR ($CDCl_3$) $\delta$:
2.03 (s, 3, $COCH_3$)
3.75 ($A_2B_2$m, 2, $O\underline{CH_2}CH_2OAc$)
4.17 ($A_2B_2$m, 2, $OCH_2\underline{CH_2}OAc$)
5.52 (s, 4, $O\underline{CH_2}$Base and $NH_2$)
7.88 (s, 1, $H_8$)
8.68 (s, 1, $H_6$)

Example 13

9-(2-acetoxyethoxymethyl)-2-acetamido-9H-purine

To a mixture of 9-(2-acetoxyethoxymethyl)-2-acetamido-6-chloro-9H-purine (3.28 g, 10 mmole) and acetone (40 ml), there were successively added triethylamine (14 ml, 100 mmole), formic acid (2 ml, 50 mmole) and 10 % palladium on charcoal (0.4 g). The mixture was heated with vigorous stirring at reflux for 1 hour, filtered while hot and the catalyst was washed with boiling acetone and chloroform. The filtrate was evaporated to dryness, the residue was dissolved in water (150 ml) and the solution was continuously extracted with dichloromethane overnight. The organic layer was dried over $Na_2SO_4$ for cca 1 hour and evaporated to dryness to give a crude product (2.78 g). It was recrystallized from 100 % ethanol to give 9-(2-acetoxyethoxymethyl)-2-acetamido-9H-purine (2.56 g, 87 %) with m.p. 134.5-135.5°C.

$^1$H NMR ($CDCl_3$) $\delta$-TMS:
2.02 (s, 3, $OCOCH_3$)
2.57 (s, 3, $NHCO\underline{CH_3}$)
3.80 ($A_2B_2$m, 2, $O\underline{CH_2}CH_2OAc$)
4.18 ($A_2B_2$m, 2, $OCH_2\underline{CH_2}OAc$)
5.66 (s, 2, $OCH_2$Base)
8.15 (s, 1, $H_8$)
9.07 (s, 1, $H_6$)
9.95 (br, 1, NH)

Example 14

2-amino-9-(2-hydroxyethoxymethyl)-9H-purine (DEOXYACYCLOVIR)

A. 9-(2-acetoxyethoxymethyl)-2-amino-9H-purine (5.02 g, 20 mmole) was dissolved in water (50 ml), 40 % aqueous methylamine (25 ml) was added and the solution was stirred at room temperature for 10 minutes. The reaction mixture was evaporated to dryness with successive addition of water and ethanol. The crude product was recrystallized from 100 % ethanol with the addition of activated charcoal to give deoxyacyclovir (3.41 g, 81.5 %) as analytically pure white crystals with m.p. 192.5-193°C.

$^1$H NMR - DMSO-$d_6$, $\delta$-DSS:
3.50 (s, 4, $CH_2CH_2$)
4.70 (s, 1, OH)
5.50 (s, 2, $OCH_2$Base)
6.63 (s, 2, $NH_2$)
8.20 (s, 1, $H_8$)
8.63 (s, 1, $H_6$)

ms m/z: 209 ($M^+$)

| Elemental analysis: | | | |
|---|---|---|---|
| Calc.: | C 45.93 | H 5.30 | N 33.47 |
| Found: | C 45.38 | H 5.19 | N 33.89 |

B. 9-(2-acetoxyethoxymethyl)-2-acetamido-9H-purine (2.05 g, 7 mmole) was dissolved in water (25 ml), 40 % aqueous methylamine (12.5 ml) was added and the solution was stirred at room temperature for 10 minutes. The reaction mixture was evaporated to dryness with successive addition of water and ethanol. The crude product was recrystallized from 100 % ethanol with the addition of activated charcoal to give deoxyacyclovir (1.16 g, 79.5 %) as white crystals, m.p. 192.5-193°C, which is identical with the product obtained in method A.

## Claims

1. $N^{2-}$acetyl-9-(2-hydroxyethoxymethyl)guanine.

2. Process for Preparing $N^2$-acetyl-9-(2-hydroxyethoxymethyl)guanine, characterized in that $N^2$-acetyl-9-(2-acetoxyethoxymethyl)guanine is treated with sodium methoxide as catalyst.

3. Compound of the formula I,

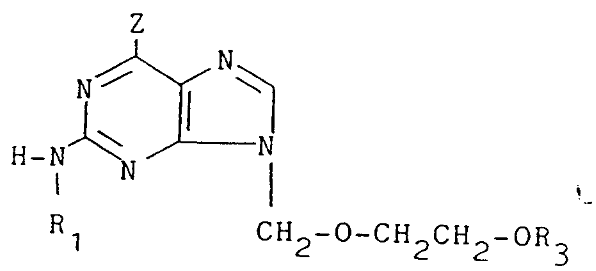

I

wherein Z = H, $R_1 = CH_3CO$ and $R_3 = H$.